Europäisches Patentamt

European Patent Office

Office européen des brevets

(11) Publication number: **0 037 378**
**B1**

(12) **EUROPEAN PATENT SPECIFICATION**

(45) Date of publication of patent specification: **12.06.85**

(21) Application number: **81810112.3**

(22) Date of filing: **23.03.81**

(51) Int. Cl.⁴: **C 08 F 220/54, A 61 K 7/11 //
(C08F220/54, 220:18, 220:06)**

(54) **Acrylic terpolymers, process for the manufacture thereof and of hair fixing formulations for use as air-pump or aerosol sprays.**

(30) Priority: **27.03.80 US 134383**

(43) Date of publication of application:
**07.10.81 Bulletin 81/40**

(45) Publication of the grant of the patent:
**12.06.85 Bulletin 85/24**

(84) Designated Contracting States:
**CH DE FR GB IT LI NL**

(56) References cited:
**GB-A-1 092 030
US-A-3 112 296
US-A-3 927 199**

(73) Proprietor: **CIBA-GEIGY AG
Postfach
CH-4002 Basel (CH)**

(72) Inventor: **Wancowicz, David J.
505 Willowbrook Run E.
Mobile Alabama 36608 (US)**
Inventor: **Lindemann, Martin K.
P.O. Box 6831
Greenville South Carolina 29606 (US)**

Courier Press, Leamington Spa, England.

### Description

Objects of the present invention are the process for the manufacture of terpolymers of acrylic acid, ethyl acrylate and *t*-butylacrylamide, the terpolymers as obtained by this process, a process for the manufacture of a hair fixing formulation containing the terpolymers, the hair fixing formulation as obtained by the process and its use as air-pump or aersol sprays.

Acrylic polymers are known in the art, e.g. in US Patent 3,112,296, to be useful as hair spray resins, viz. as polymers which are suitable for use as hair fixing formulations in e.g. air-pump sprays or aerosol cans. Among the particularly useful acrylic polymers is the terpolymer acrylic acid (AA)—ethyl acrylate (EA)—*t*-butylacrylamide (tBAM).

In Example 1 of US Patent 3,112,296, for instance, the terpolymer AA-EA-tBAM is commonly made by copolymerizing ethyl acrylate and *t*-butylacrylamide and then partially saponifying the ethyl acrylate moieties in the polymer chain to give acrylic acid moieties. This method is now recognized as giving a terpolymer having less than the ideal balance of water solubility and curl retention properties of the final resin, probably due to an uneven distribution of carboxylate groups down the polymer chain.

A second method of manufacture of a terpolymer, having AA-EA units therein, is the terpolymerization of acrylic acid, ethyl acrylate and a third acrylic monomer. But by methods known to the art as e.g. described in US Patent 3,927,199 for acrylic acid, methyl acrylate and various acrylic monomers as third monomers, these terpolymerizations are not wholly satisfactory in giving a hair spray resin having balanced water solubility and hair curl retention properties. Reproducibility from batch to batch of terpolymerization runs also are a problem. Finally, this method as well as the copolymer saponification method result in a broad range of polymer chain lengths in the final terpolymer resin, together with appreciable amounts of unreacted monomers that contribute undesirable odors and toxicity to the final product.

It is highly desirable that a hair spray resin be water soluble for easy removal from the hair by shampooing. This need for water solubility must be balanced against a certain degree of hydrophobicity needed for curl retention. As can be appreciated, curl retention is adversely affected by humidity and high moisture content of the hair. In a humid atmosphere, the moisture content of the hair is generally enhanced by hydrophilic resins on the hair and decreased by hydrophobic resins on the hair.

It has now been found that a particularly desirable balance of water solubility and curl retention properties can be achieved by an AA-EA-tBAM terpolymer which has the carboxylate groups evenly and uniformly distributed along the polymer chain.

It is also desirable in a hair spray resin of this type to have an average molecular weight of about 15000 to about 60000, with a very narrow distribution of polymer chain lengths within this range.

It is an object of this invention to provide an AA-EA-tBAM terpolymer of the above indicated kind for use as a hair spray resin, which the advantageous properties as described above having in contrast to polymers manufactured by known methods substantially no unreacted monomer present in the terpolymer and that particularly shows solution clarity in water and alcohol, and is above all reproducibly manufacturable under defined conditions.

The objects of this invention are realized according to the present invention by a new process for the manufacture of a polymer of acrylic acid, ethyl acrylate and *t*-butylacrylamide which is characterized in that the polymerization process comprises the steps of

(a) preparing an ethanol solution of a monomer mixture of about 6 to about 8 percent by weight of acrylic acid, about 37 to about 45 percent by weight of ethyl acrylate and about 46 to about 56 percent by weight of *t*-butylacrylamide, the indicated percentages referring to the total monomers composition,

(b) introducing in a first portion 5 to 15, in particular about 10 percent of the total weight of the monomer mixture solution as prepared in step (a) into a reaction vessel containing refluxing ethanol; and then

(c) without further heating, continuously and substantially simultaneously adding the remainder, viz. the second portion of the monomer mixture solution and an ethanol solution of a free-radical generating polymerization initiator to the reaction vessel over a period of about 1 to about 3 hours at a rate that maintains reflux of the reaction mixture by the heat of the polymerization reaction, provided that the addition of the monomer mixture solution is completed about 15 to about 30 minutes before the addition of the initiator solution is completed.

The monomer mixture as prepared in step (a) of the inventive process preferably contains about 7 percent by weight of acid, about 41 percent by weight of ethyl acrylate and about 51 percent by weight of *t*-butylacrylamide. The monomer mixture solution optionally contains a minor effective amount, e.g. about 0.001 to about 0.05 percent by weight, of a polymerization inhibitor as is commonly provided in commercial samples of acrylic monomers. Such polymerization inhibitors are well known in the art, and are generally hydroquinone or hydroquinone derivatives, particularly the monomethyl ether of hydroquinone.

In a preferred embodiment, the ethanol solution of the monomer mixture also contains water, so that the ethanol used in step (a) in the monomer mixture solution contains about 30 to about 40, in particular about 35 percent by weight of water, the water being supplied by the water content of the monomer and/or by direct addition of water. It is preferred that all of the water is supplied by the water content of the monomer, in particular of t-BAM, which thus is used in this particular embodiment as an aqueous paste. An amount of ethanol and water is generally applied in order to obtain a mixture containing preferably about 35 to about 45, in particular about 40 percent by weight of total monomers.

Any free radical polymerization initiator which is commonly used in the polymerization art comes generally into consideration as initiator for use in step (c) of the inventive process. Organic peroxide initiators are however preferred.

It is crucial to the performance of the inventive terpolymerization process that the polymerization initiator be added in step (c) to the reaction mixture in solution, preferably in alcohol solution. Non-peroxide initiator, e.g. 2,2' - azobis(2,4' - dimethylvaleronitrile) can be used successfully in alcohol solution, but not as successfully as the preferred peroxide initiator. As the artisan will appreciate, the rate of addition of the monomer mixture solution can be adjusted to accommodate different activities of different polymerization initiators. Suitable peroxy initiators appear to be those having a half-life of about one hour at a temperature in the range of about 70—120°C. Commercially available alcohol-soluble, organic peroxide polymerization initiators include t-butylperbenzoate, t-butylperoxyneodecanoate, 2,5 - dimethyl - 2,5' - bis(2 - ethylhexanoylperoxy)hexane, O,O - t - butyl - O - isopropylmonoperoxy carbonate, and t-butylperoctoate. Of these, t-butylperoctoate is preferred.

A further crucial feature of the inventive process for the manufacture of the terpolymer is the sequence and time of addition of the monomer mixture solution and of the initiator solution as indicated above in step (c) since early polymerization in step (a) leads to a polymer of excessively high molecular weight and vapor-phase polymerization of the refluxate in step (c) leads to resins that are cloudy in water and show poor washability, due to the formation of a predominantly ethyl acrylate polymer. Both of these undesirable effects are avoided by the method as described in the inventive process. In a particularly preferred embodiment of the inventive process, the initiator solution is added over a period of about 2.0 to about 2.5 hours and the monomer mixture solution is added over a period of about 1.5 to about 2.0 hours in step (c). Increasing the initiator addition time or decreasing the monomer addition time, increases the molecular weight.

The weight ratio of the initiator to the total monomers can vary in a relative wide range. It is however preferred that the weight ratio in process step (c) of total monomers to polymerization initiator is about 100:1 to about 125:1.

It is advantageous to carry out step (a) under an atmosphere of an inert gas which contains about 2.5 to about 10 percent by volume of oxygen, preferably about 6 percent per volume, and steps (b) and (c) under an inert gas atmosphere. In both cases, the inert gas is preferably nitrogen.

The terpolymers as obtained by the inventive process for their manufacture are characterized by their molecular weight of about 15000 to about 60000 and their narrow range of polymer chain length distribution as stated above, that is a dispersity up to 2.5.

Dispersity is the ratio of the weight-average and number-average molecular weights, and is a measure of the molecular weight distribution; the greater the dispersity, the broader the distribution range (also see "Die Synthese von einheitlichen Polymeren" by J. H. Winter, Springer-Verlag Berlin Heidelberg New York 1967, page 29).

Molecular weights and dispersities can be determined by gel permeation chromatography (=GPC) according to known methods. See e.g. Bulletin "Calibration of GPC Systems" published April, 1974, by Waters Associates, Inc., Maple Street, Milford, Massachusetts 01757, "Gel Permeation Chromatography, A new Method for Molecular Weight Distribution of High Polymers" by J. C. Moore in J. of Polymer Sciences, Part A, Vol. 2, p. 835 to 843 (1964) and "Application of Gel Permeation Chromatography to High and Low Molecular Weight Polymers", by L. F. Maley in J. of Polymer Sciences, Part C, Vol. 8, p. 253 to 268 (1965).

Preferred terpolymers have molecular weights of about 25000 to about 35000, most preferably about 30000 and a dispersity not greater than about .1.5 to about 2.5, preferably not greater than about 2.0.

Such terpolymers can be incorporated in hair fixing formulations by methods which themselves are well-known in the art. The process for the manufacture of hair fixing composition for this purpose is characterized in that the carboxylic groups of the polymer in ethanol solution as inventively obtained are neutralized by titration with a base, particularly with potassium hydroxide, preferably an aqueous solution of potassium hydroxide of a preferred strength of 40 percent by weight and the polymer in ethanolic solution as neutralized in this way is then further diluted with ethanol to a polymer content of about 0.2 to about 10, preferably about 2.5 percent by weight.

Optional additives may be incorporated into the hair fixing formulations of this invention in order to modify certain properties thereof. Among these additives may be included: plasticizers such as glycols, phthalate esters and glycerine, silicones, emollients, lubricants and penetrants such as lanolin compounds, protein hydrolyzates and other protein derivatives, ethylene oxide adducts and polyoxyethylene cholesterol, U.V. absorbers, dyes and other colorants and perfumes. Total amount of these ingredients does not exceed about 10 percent by weight of the weight of the terpolymer used in the formulation. The resins of this invention show little or no tendency to chemically interact with such additives.

The hair fixing formulations as obtained by the above described process for their manufacture are characterized in that they contain about 0.2 to about 10 percent by weight of the neutralized polymer and optionally usual hair cosmetic additives as above described, the formulation being preferably an ethanol solution.

Such hair fixing formulations are suitable for use as air-pump sprays or as sprays charged to butan/propane-propellent aerosol cans. When charged in such pump sprays and aerosol cans and used as a hair spray, the inventive hair fixing formulations exhibit all of the characteristics required of such a product. Their films are transparent, glossy, flexible and strong. They possess good antistatic properties, adhere well to hair, are easily removed by soapy water or shampoos, allow the hair to be readily recombed, do not yellow on aging, do not become tacky when exposed to high humidities and have excellent curl retention even under high humidity conditions.

Standard hair-spray formulations and use-evaluation methods are set out in "New Concepts in Hair Products" by Mel Hoyt in Cosmetics and Perfumery, Vol. 88, p. 53 to 55 (1973).

The above-described invention is further set out below in the following examples, wherein all parts and percents are by weight unless otherwise specified:

Example 1

A solution is prepared and stored, having 76 parts of acrylic acid, 422 parts of ethyl acrylate and 528 parts of t-butylacrylamide dissolved in 528 parts of water and 1001 parts of ethanol. The water is derived from using 1056 parts of a 50% paste of t-butylacrylamide. The EA and AA monomers, being commercial samples, contain polymerization-inhibiting amounts of the monomethylether of hydroquinone, generally about 0.01 to about 0.02 percent by weight. The solution is prepared and stored under a nitrogen atmosphere, containing about 6 percent by volume of oxygen.

A second solution is prepared, having 8.9 parts of t-butylperoctoate dissolved in 77 parts of ethanol.

About 10% of the first solution is charged to a reaction vessel, equipped with a reflux condenser and a gate agitator, under a nitrogen atmosphere. At the time of the charge, the reaction vessel contains 144 parts of refluxing ethanol. External heating is discontinued while separate streams of the second solution and the remaining 90% of the first solution are introduced into the reaction vessel at a rate to maintain reflux, over a period of about 1.5 to about 2.5 hours. The addition of all of the first (monomer mixture) solution is completed about 15 minutes to about 1/2 hour before completion of the second (initiator) solution. The initiator solution is added over a period of about 2.0 to 2.5 hours and the monomer mixture solution over a period of about 1.5 to 2.0 hours.

Agitation under nitrogen atmosphere is continued, after completion of the addition of the last of the initiator solution, until the reaction is complete, about 2 hours.

Finally solvent is distilled off under a nitrogen stream till a concentration of about 50 percent solids is reached.

The terpolymer of this Example can be used as the alcohol solution, or all or part of the alcohol can be removed by distillation.

The terpolymer of this Example has a weight average molecular weight of about 30000 with a dispersity of about 2.0.

Examples 2 and 3

Repeated runs of Example 1 are made in Examples 2 and 3.

Characteristics of polymers of Examples 1 to 3

The terpolymers of Examples 1 to 3 show the following characteristics:

| Properties | Example 1 | Example 2 | Example 3 |
|---|---|---|---|
| Average molecular weight** | 30,026 | 27,328 | 27,107 |
| Dispersity** | 1.98 | 1.85 | 1.85 |
| Unreacted monomer (p.p.m.) | N.D.* | N.D.* | N.D.* |
| Water solution | clear | clear | clear |
| Alcohol solution | clear | clear | clear |
| Odor | alcohol | alcohol | alcohol |

*N.D.: none detected; detectable at 10 p.p.m.
**determined by GPC according to "Calibration of GPC Systems" of April, 1974, by Water Associates.

The comparison of the characteristics of the terpolymers of Example 1 to 3 shows that a dispersity of about 2, that is 1.5 to 2.5 and an average molecular weight of about 2500 to about 35000 are thus readily achieved.

Example 4

The alcohol solution of the resin obtained in Example 1 is titrated to neutrality with 40% aqueous potassium hydroxide and further diluted with ethanol to give a solid content of 2.5%. The resulting dilute, neutral solution of the polymer is charged as a hair fixing formulation to an air-pump spray or to a butane/propane-propellant aerosol can for use as a hair spray, whereby the good properties of the hair fixing formulations as described above are performed.

## Claims

1. Process for the manufacture of a polymer of acrylic acid, ethyl acrylate and t-butylacrylamide, characterized in that the polymerization process comprises the steps of

(a) preparing an ethanol solution of a monomer mixture of 6 to 8 percent by weight of acrylic acid, 37 to 45 percent by weight of ethyl acrylate and 46 to 56 percent by weight of t-butylacrylamide, the percentages referring to the total monomers composition,

(b) introducing a first portion of 5 to 15 percent of the total weight of the monomer mixture solution into a reaction vessel containing refluxing ethanol, and then

(c) without further heating, continuously and substantially simultaneously adding the remainder second portion of the monomer mixture solution and and ethanol solution of a free-radical generating polymerization initiator to the reaction vessel over a period of 1 to 3 hours at a rate that maintains reflux of the reaction mixture by the heat of the polymerization reaction, provided that the addition of the monomer mixture solution is completed 15 to 30 minutes before the addition of the initiator solution is completed.

2. Process according to claim 1, characterized in that the ethanol used in step (a) in the monomer mixture solution contains 30 to 40 percent by weight of water, the water being supplied by the water content of the monomers and/or by direct addition of water.

3. Process according to claim 1, characterized in that an organic peroxide is used as polymerization initiator in step (c).

4. Process according to claim 1, characterized in that the initiator solution is added over a period of 2.0 to 2.5 hours and the monomer mixture solution is added over a period of 1.5 to 2.0 hours in step (c).

5. Process according to claim 1, characterized in that the weight ratio in step (c) of total monomers to polymerization initiator is 100:1 to 125:1.

6. Process according to claim 1, characterized in that steps (a), (b) and (c) are carried out under an atmosphere of inert gas which in step (a) further contains 2.5 to 10 percent by volume of oxygen.

7. Polymer as obtained by the process according to any one of claims 1 to 6, characterized in that its average molecular weight is 15000 to 60000 and its dispersity is up to 2.5.

8. Process for the manufacture of a hair fixing formulation, characterized in that the carboxylic groups of the polymer in ethanol solution as obtained in claim 7 are neutralized by titration with an aqueous potassium hydroxide solution and the polymer is then diluted with ethanol to a polymer content of 0.2 to 10 percent by weight.

9. Hair fixing formulation as obtained by the process according to claim 8, characterized in that it contains 0.2 to 10 percent by weight of the neutralized polymer and optionally usual hair cosmetic additives.

10. Use of the hair fixing formulation according to claim 9 as air-pump spray or as a spray charged to a butane/propane-propellant aerosol can.

## Patentansprüche

1. Verfahren zur Herstellung von Polymeren aus Acrylsäure, Ethylacrylat und tert-Butylacrylamid, dadurch gekennzeichnet, dass das Polymerisationsverfahren die folgenden Stufen (a), (b) und (c) umfasst:

(a) Herstellung einer Lösung in Ethanol eines Monomergemisches aus 6 bis 8 Gewichtsprozent Acrylsäure, 37 bis 45 Gewichtsprozent Ethylacrylate und 46 bis 56 Gewichtsprozent tert-Butylacrylamid, wobei sich die Prozentangaben auf das Gesamtgewicht der Monomerzusammensetzung beziehen.

(b) Zugabe einer ersten Portion von 5 bis 15 Prozent, bezogen auf das Gesamtgewicht der Monomergemischlösung in ein Reaktionsgefäss, das Ethanol unter Rückfluss enthält und

(c) anschliessende, kontinuierlich Zugabe ohne weitere Wärmezufuhr der verbleibenden zweiten Portion der Monomergemischlösung unter möglichst gleichzeitiger Zugabe einer Lösung in Ethanol eines freie Radikale bildenden Polymerisationsinitiators in das Reaktionsgefäss innerhalb einer Zulaufszeit von 1 bis 3 Stunden, welche den Rückfluss des Reaktionsgemisches durch die Reaktionswärme der Polymerisation bewerkstelligt, mit der Massgabe, dass die Zugabe der Monomergemischlösung 15 bis 30 Minuten vor der Zugabe der Initiatorlösung beendet wird.

2. Verfahren nach Anspruch 1, dadurch gekennzeichnet, dass das in Stufe (a) eingesetzte Ethanol zum lösen des Monomergemisches 30 bis 40 Gewichtsprozent Wasser enthält, wobei das Wasser als Wassergehalt der Monomeren und/oder durch direkte Wasserzugabe zugeführt wird.

3. Verfahren nach Anspruch 1, dadurch gekennzeichnet, dass man als Polymerisationsinitiator in Stufe (c) ein organisches Peroxid einsetzt.

4. Verfahren nach Anspruch 1, dadurch gekennzeichnet, dass man die Initiatorlösung innerhalb von 2,0 bis 2,5 Stunden und die Monomergemischlösung innerhalb von 1,5 bis 2,0 Stunden zugibt.

5 Verfahren nach Anspruch 1, dadurch gekennzeichnet, dass das Gewichtsverhältnis Monomergesamtgewicht:Polymerisationsinitiator 100:1 bis 125:1 beträgt.

6. Verfahren nach Anspruch 1, dadurch gekennzeichnet, dass man die Stuffen (a), (b) und (c) in Inertgas durchführt, wobei das Gas in Stufe (a) zusätzlich 2,5 bis 10 Volumenprozent Sauerstoff enthält.

7. Das nach dem Verfahren gemäss einem der Ansprüche 1 bis 6 hergestellte Polymer, dadurch gekennzeichnet, dass es ein mittleres Molekulargewicht von 15000 bis 60000 und eine Dispersität von höchstens 2,5 aufweist.

8. Verfahren zur Herstellung einer Harrfestigungsmittel-Formulierung, dadurch gekennzeichnet, dass man die Carboxylgruppe des in Ethanol gelösten Polymers gemäss Anspruch 7 durch Titration mit einer wässrigen Kaliumhydroxidlösung neutralisiert und anschliessend das Polymer mit Ethanol auf ein Gehalt von 0,2 bis 10 Gewichtsprozent verdünnt.

9. Die nach dem Verfahren gemäss Anspruch 8 hergestellte Haarfestigungsmittel-Formulierung, dadurch gekennzeichnet, dass sie 0,2 bis 10 Gewichtsprozent des neutralisiertem Polymers und gegebenenfalls übliche, kosmetische Zusätze enthält.

10. Verwendung der Haarfestigungsmittel-Formulierung gemäss Anspruch 9 als Luftpumpe- oder Aerosolzerstäuber, wobei das Aerosol ein Butan/Propangemisch als Treibmittel enthält.

**Revendications**

1. Procédé pour la fabrication d'un polymère d'acide acrylique, acrylate d'éthyle et *t*-butylacrylamide, caractérisé par le fait que le procédé de polymérisation comprend les stades suivants:

(a) préparation d'un solution dans l'éthanol d'un mélange de monomères de 6 à 8% en poids d'acide acrylique, 37 à 45% en poids d'acrylate d'éthyle et 46 à 56% en poids de *t*-butylacrylamide, les pourcentages se rapportant à l'ensemble des monomères de la composition,

(b) introduction d'une première portion de 5 à 15% du poids total de la solution du mélange de monomères dans un récipient laboratoire contenant de l'éthanol bouillant au reflux; puis

(c) sand chauffage ultérieur, addition en continue et sensiblement simultanée de la seconde portion restante de la solution du mélange de monomères et d'une solution éthanolique d'un amorceur de polymérisation engendrant des radicaux libres dans le récipient laboratoire en l'espace de 1 à 3 heures à une vitesse qui maintienne le reflux du mélange réactionnel par la chaleur de la réaction de polymérisation, et en faisant en sorte que l'addition de la solution du mélange de monomères soit terminée 15 à 30 minutes avant la fin de l'addition de la solution de l'amorceur.

2. Procédé selon la revendication 1, caractérisé par le fait que l'éthanol utilisé dans le stade (a) dans la solution du mélange de monomères contient 30 à 40% en poids d'eau, l'eau étant forunie par la teneur en eau des monomères et/ou par addition directe d'eau.

3. Procédé selon la revendication 1, caractérisé par le fait que, dans le stade (c), un peroxyde organique est utilisé comme amorceur de polymérisation.

4. Procédé selon la revendication 1, caractérisé par le fait que, dans le stade (c), la solution d'amorceur est ajoutée en l'espace de 2 à 2,5 heures et la solution du mélange de monomères est ajoutée en l'espace de 1,5 à 2 heures.

5. Procédé selon la revendication 1, caractérisé par le fait que, dans le stade (c), le rapport pondéral du total des monomères à l'amorceur de polymérisation est de 100:1 à 125:1.

6. Procédé selon la revendication 1, caractérisé par le fait que les stades (a), (b) et (c) sont effectués sous une atmosphère d'un gaz inerte qui, dans le stade (a), contient en plus 2,5 à 10% en volume d'oxygène.

7. Polymère ainsi obtenu par le procédé selon l'une quelconque des revendications 1 à 6, caractérisé par le fait que son poids moléculaire moyen est de 15 000 à 60 000 et que sa dispersité va jusqu'à 2,5.

8. Procédé de fabrication d'une composition de laques pour cheveux, caractérisé par le fait que les groupes carboxyliques du polymère en solution éthanolique tel qu'obtenu dans la revendication 7, sont neutralisés par titrage avec une solution aqueuse d'hydroxyde de potassium puis que le polymère est dilué avec de l'éthanol pour avoir une teneur en polymère de 0,2 à 10% en poids.

9. Composition de laques pour cheveux ainsi obtenue par le procédé selon la revendication 8, caractérisée par le fait qu'elle contient 0,2 à 10% en poids du polymère neutralisé et facultativement des additifs classiques cosmétiques capillaires.

10. Utilisation de la composition de laques pour cheveux selon la revendication 9, comme produit à pulvériser par pompe à air ou comme produit à pulvériser chargé dans un emballage métallique pour aérosol avec propellents butane/propane.